# EUROPEAN PATENT SPECIFICATION

(11) **EP 3 604 503 B1**
(45) Date of publication and mention of the grant of the patent: **18.03.2026**
(21) Application number: 18778261.0
(22) Date of filing: 27.03.2018
(51) Int. Cl.: C12N 5/10, C12N 5/02, C12N 5/0735, C12N 5/074

(54) **ADDITIVE FOR UNDIFFERENTIATION MAINTAINING MEDIUM**
ADDITIV FÜR UNDIFFERENZIERUNGSERHALTUNGSMEDIUM
ADDITIF POUR MILIEU DE MAINTIEN DE DÉDIFFÉRENCIATION

(30) Priority: 28.03.2017 JP 2017063842
(43) Date of publication of application: 05.02.2020
(73) Proprietor: AJINOMOTO CO., INC., Chuo-ku Tokyo 104-8315 (JP); Keio University, Tokyo 108-8345 (JP)
(72) Inventor: KOSEKI, Kotoe, Kawasaki-shi Kanagawa 210-8681 (JP); OKAMOTO, Satoru, Kawasaki-shi Kanagawa 210-8681 (JP); TOHYAMA, Shugo, Tokyo 160-8582 (JP); FUJITA, Jun, Tokyo 160-8582 (JP); FUKUDA, Keiichi, Tokyo 160-8582 (JP); SOMEYA, Shota, Tokyo 160-8582 (JP)
(74) Representative: Hoffmann Eitle
(86) International application number: PCT/JP2018/012476
(87) International publication number: WO 2018/181342

(56) References cited:
- EP-A1- 2 218 778
- WO-A1-2012/056997
- WO-A1-2013/147264
- WO-A1-2015/182140
- WO-A1-2016/010165
- WO-A1-2016/027850
- WO-A1-90/03430
- CN-A- 104 357 379
- JP-A- 2010 534 072
- JP-A- 2013 524 817
- JP-A- 2014 520 535
- JP-A- 2016 190 842
- JP-A- 2016 501 026
- SALAZAR ANDREW ET AL: "Amino acids in the cultivation of mammalian cells", AMINO ACIDS, SPRINGER VERLAG, AU, vol. 48, no. 5, 1 February 2016 (2016-02-01), pages 1161 - 1171, XP035889636, ISSN: 0939-4451, [retrieved on 20160201], DOI: 10.1007/S00726-016-2181-8
- JONES ET AL: "The Kynurenine Pathway in Stem Cell Biology", INTERNATIONAL JOURNAL OF TRYPTOPHAN RESEARCH, vol. 6, 1 September 2013 (2013-09-01), pages 57 - 66, XP055240814, DOI: 10.4137/IJTR.S12626

## Description

### [Technical Field]

The present invention relates to the use of a serum-free medium for efficiently proliferating pluripotent stem cells while maintaining an undifferentiated state, a method for proliferating pluripotent stem cells in the medium, and the like.

### [Background Art]

Pluripotent stem cells such as ES cell (Embryonic stem cell), induced pluripotent stem cell (iPS) and the like are expected to be usable in regenerative medicine and the like because of their superior proliferation capacity and multipotency. In particular, iPS cell is regarded as a highly superior material for regenerative medicine in view of the facts that it is produced and acquired relatively easily, only a few ethical restrictions are imposed on the production, and the aspect of rejection in transplantation.

When regenerative medicine is performed using pluripotent stem cells, a large amount of pluripotent stem cells are required for the treatment of diseases and the development and research of treatment methods. Thus, it is important to develop and improve a method for culturing pluripotent stem cells that can supply a large amount of pluripotent stem cells. Above all, it is essential to improve the culture medium. Culturing a large amount of cells requires a large amount of medium. For example, when 10⁶ iPS cells are cultured to produce about 10¹⁰ myocardial cells which are transplanted to a single patient, 100 liters of a medium is required per patient, and the cost of the medium will reach about USD 100,000 at the lowest estimate. One way to reduce the cost of the medium is to increase the number of cells that can be cultured per unit medium volume. That is, there is a need for a more highly efficient and low-cost effective medium for culturing pluripotent stem cells.

Generally, essential amino acids are added to the medium. For example, mTeSR1 medium (non-patent documents 1, 2, 3) and Essential-8 medium (non-patent document 4), which are media for culturing stem cells, are based on Dulbecco's modified Eagle medium (DMEM)/F12 medium and added with some factors such as bFGF, insulin and the like. The content of amino acids in the DMEM/F12 medium is set based on the amount of free amino acids in blood, and 9.0200 mg/L of L-tryptophan is contained therein. While many media have been developed so far, the amino acid composition in the media has hardly been improved.

Salazar et al. describes amino acids in the cultivation of mammalian cells and indicates a concentration of L-Trypthophan in CCM of between 0.005 and 0.080 g/L (Salazar, Andrew, Michael Keusgen, and Jörg Von Hagen. "Amino acids in the cultivation of mammalian cells." Amino acids 48.5 (2016): 1161-1171).

WO90/03430 describes protein-free cell culture media supplements and indicates that Tryptophan is recognized as an essential amino acid and is included in typical serum-supplemented and serum-free media at 2-20 mg/L.

WO 2009/014272 relates to a method for the preparation of dermal papilla tissue using a medium for culturing mesenchymal stem cells.

### [Document List]

### [Patent documents]

patent document 1: JP-A-2004-135672
patent document 2: JP-A-2007-228815
patent document 3: US2010/0317104A1
patent document 4: WO2011/100286A2

### [non-patent documents]

non-patent document 1: Ludwig TE et. al. Nat. Methods 3(8):637-46; 2006
non-patent document 2: Ludwig TE et. al. Nat. Biotechnol 24(2):185-7; 2006
non-patent document 3: Masters et. al. Human Cell Culture. Dordrecht: Springer Netherlands; 2007
non-patent document 4: Chen G et. al. Nat. Methods 8(5)424-9; 2011

### [SUMMARY OF THE INVENTION]

### [Problems to be Solved by the Invention]

An object of the present invention is to provide a medium capable of efficiently proliferating pluripotent stem cells and a method for efficiently proliferating pluripotent stem cells using the medium.

### [Means of Solving the Problems]

To achieve the above-mentioned object, the present inventors took note of changes in the amount of amino acids in the medium during the culture process of pluripotent stem cells. As a result, they have found that the amount of L-tryptophan in the medium rapidly decreases during the culture process of pluripotent stem cells, and L-tryptophan depletes earliest among all amino acids contained in the medium. Here, the medium formulation concentration of L-tryptophan, which is an essential amino acid, is lower than that of other amino acids (non-patent documents 1, 2, 3, 4), and it is predicted that L-tryptophan will run out quickly during mass proliferation of pluripotent stem cells and become a limiting factor for cell proliferation. The present inventors have found that proliferation of pluripotent stem cells can be promoted by increasing the amount of L-tryptophan in the medium or supplementing the consumed L-tryptophan by adding L-tryptophan during the culture, which resulted in the completion of the present invention.

That is, the present invention provides the following.
[1] Use of a serum free medium for culturing a pluripotent stem cell, wherein the medium comprises L-tryptophan or an L-tryptophan derivative at a concentration of not less than 176 µM.
[2] The use of a medium of [1] wherein the concentration of the L-tryptophan or L-tryptophan derivative in the medium is 176 µM - 1408 µM.
[3] The use of the medium of [1] or [2] wherein the medium is a serum-free medium.
[4] The use of the medium of any of [1] to [3] wherein the pluripotent stem cell is an induced pluripotent stem cell.
[5] The use of the medium of any of [1] to [4] wherein the tryptophan derivative is a dipeptide in which tryptophan and amino acid are peptide-bonded.
[6] The use of the medium of [5] wherein the dipeptide is L-alanyl-L-tryptophan.
[7] A method for culturing a pluripotent stem cell comprising culturing the pluripotent stem cell in the medium of any of [1] to [6].
[8] The method of [7] wherein the method is for proliferating a pluripotent stem cell.
[9] A pluripotent stem cell culture preparation comprising the medium of any of [1] to [6] and a pluripotent stem cell.
[10] A method for culturing a pluripotent stem cell comprising the following steps:
   (1) culturing a pluripotent stem cell in a medium comprising L-tryptophan or an L-tryptophan derivative;
   (2) adding L-tryptophan or an L-tryptophan derivative to the obtained pluripotent stem cell culture such that the concentration of the L-tryptophan or an L-tryptophan derivative in the medium is not less than 176 µM to supplement a part or all of the L-tryptophan or L-tryptophan derivative in the medium and consumed in (1); and
   (3) successively culturing the pluripotent stem cell culture added with the L-tryptophan or L-tryptophan derivative.
[11] The method of [10] wherein the medium comprising the L-tryptophan or L-tryptophan derivative is a serum-free medium.
[12] The method of [10] or [11] wherein the tryptophan derivative is a dipeptide in which tryptophan and amino acid are peptide-bonded.
[13] The method of [12] wherein the dipeptide is L-alanyl-L-trypotophan.
[14] Use of a serum-free medium additive for promoting proliferation of a pluripotent stem cell comprising L-tryptophan or an L-tryptophan derivate, wherein the concentration of the L-tryptophan or an L-tryptophan derivative in the medium is not less than 176 µM.
[15] The use of a medium of additive of [14] wherein the tryptophan derivative is a dipeptide in which tryptophan and amino acid are peptide-bonded.
[16] The use of the medium additive of [15] wherein the dipeptide is **L-**alanyl-L-tryptophan.

### [Effect of the Invention]

According to the present invention, proliferation of pluripotent stem cells can be promoted, and thus pluripotent stem cells can be efficiently cultured in large amounts. Expected specific effects of using this medium are that the target number of cells can be obtained in a short period of time compared to conventional media, the target number of cells can be obtained without changing to culture facility specialized for mass culture but using the existing facility, and the like. Therefore, human costs and monetary costs for culturing pluripotent stem cells can be drastically reduced.

### [Brief Description of the Drawings]

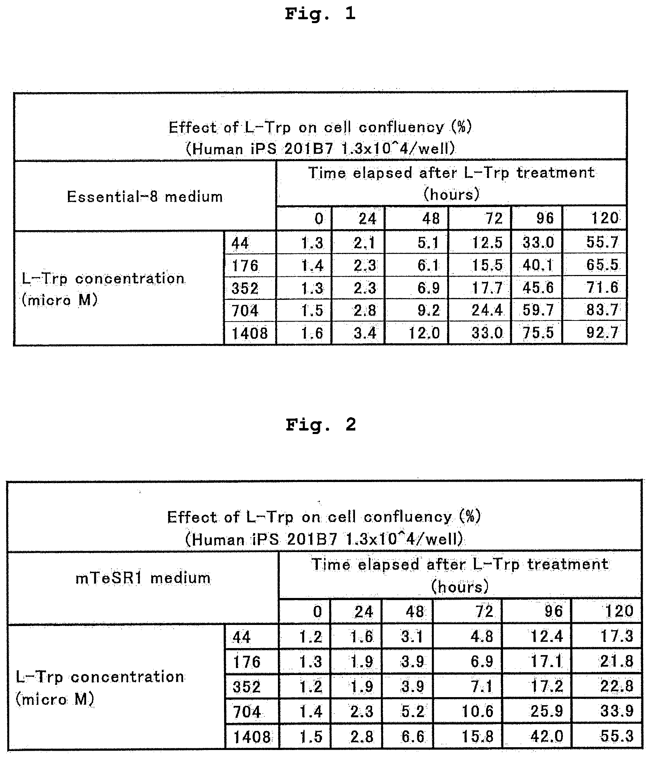

Fig. 1 shows the cell coating rate (%) of human induced pluripotent stem cells 201B7 in Essential-8 medium supplemented with L-tryptophan to a final concentration of 44 µM, 176 µM, 352 µM, 704 µM or 1408 µM. 201B7 cells were single cell seeded in a 6-well plate at 13,000 cells per well and cultured for 6 days. The cell coating rate was measured at 24, 48, 72, 96 and 120 hr after addition of L-tryptophan to the above-mentioned concentrations (0 hr). The obtained results showed an **L-**tryptophan concentration-dependent effect of promoting proliferation.
Fig. 2 shows the cell coating rate (%) of human induced pluripotent stem cells 201B7 in mTeSR1 medium supplemented with L-tryptophan to a final concentration of 44 µM, 176 µM, 352 µM, 704 µM or 1408 µM. 201B7 cells were single cell seeded in a 6-well plate at 13,000 cells per well and cultured for 6 days. The cell coating rate was measured at 24, 48, 72, 96 and 120 hr after addition of L-tryptophan to the above-mentioned concentrations (0 hr). The obtained results showed an L-tryptophan concentration-dependent effect of promoting proliferation.

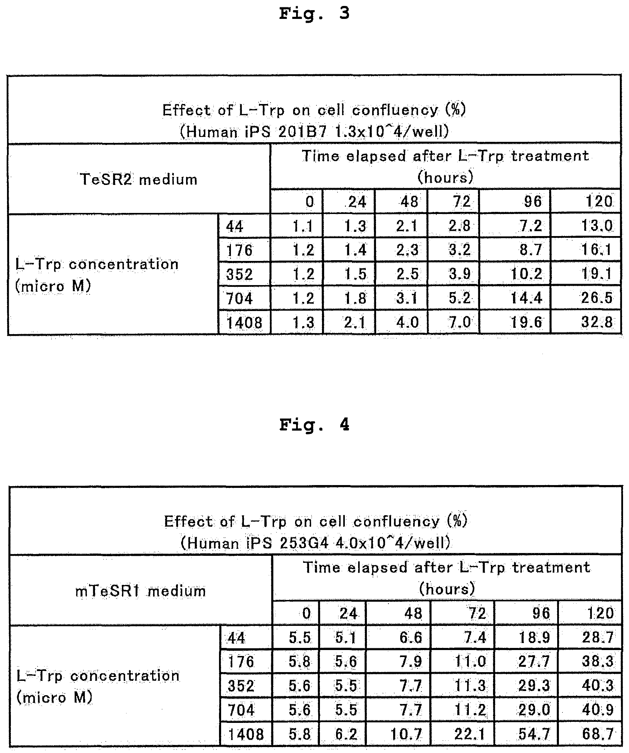

Fig. 3 shows the cell coating rate (%) of human induced pluripotent stem cells 201B7 in TeSR2 medium supplemented with L-tryptophan to a final concentration of 44 µM, 176 µM, 352 µM, 704 µM or 1408 µM. 201B7 cells were single cell seeded in a 6-well plate at 13,000 cells per well and cultured for 6 days. The cell coating rate was measured at 24, 48, 72, 96 and 120 hr after addition of L-tryptophan to the above-mentioned concentrations (0 hr). The obtained results showed an L-tryptophan concentration-dependent effect of promoting proliferation.
Fig. 4 shows the cell coating rate (%) of human induced pluripotent stem cells 253G4 in mTeSR1 medium supplemented with L-tryptophan to a final concentration of 44 µM, 176 µM, 352 µM, 704 µM or 1408 µM. 253G4 cells were single cell seeded in a 6-well plate at 40,000 cells per well and cultured for 6 days. The cell coating rate was measured at 24, 48, 72, 96 and 120 hr after addition of L-tryptophan to the above-mentioned concentrations (0 hr). The obtained results showed an L-tryptophan concentration-dependent effect of promoting proliferation.

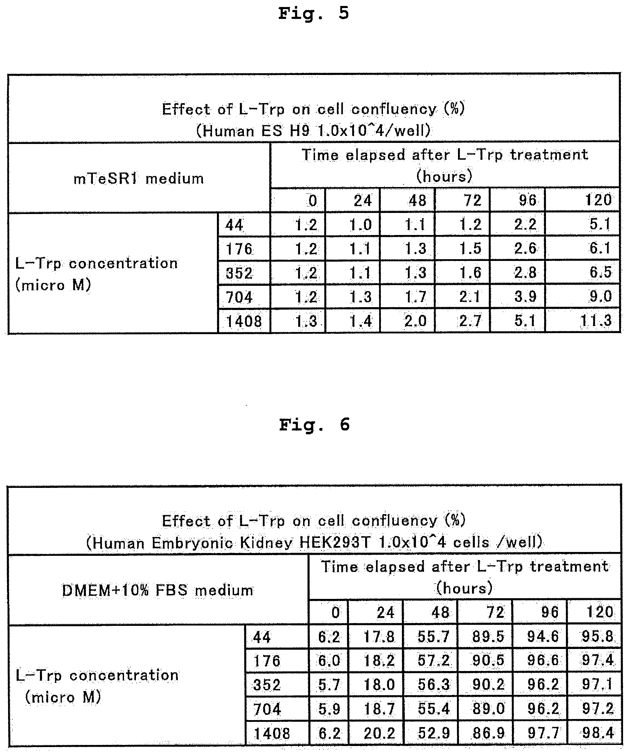

Fig. 5 shows the cell coating rate (%) of human embryonic stem cells H9 in mTeSR1 medium supplemented with L-tryptophan to a final concentration of 44 µM, 176 µM, 352 µM, 704 µM or 1408 µM. H9 cells were single cell seeded in a 6-well plate at 10,000 cells per well and cultured for 6 days. The cell coating rate was measured at 24, 48, 72, 96 and 120 hr after addition of L-tryptophan to the above-mentioned concentrations (0 hr). The obtained results showed an L-tryptophan concentration-dependent effect of promoting proliferation.
Fig. 6 shows the cell coating rate (%) of human embryonic kidney cell 293T in 10% FCS-added DMEM medium supplemented with L-tryptophan to a final concentration of 44 µM, 176 µM, 352 µM, 704 µM or 1408 µM. 293T cells were single cell seeded in a 6-well plate at 10,000 cells per well and cultured for 6 days. The cell coating rate was measured at 24, 48, 72, 96 and 120 hr after addition of L-tryptophan to the above-mentioned concentrations (0 hr). The obtained results did not show an L-tryptophan concentration-dependent effect of promoting proliferation.

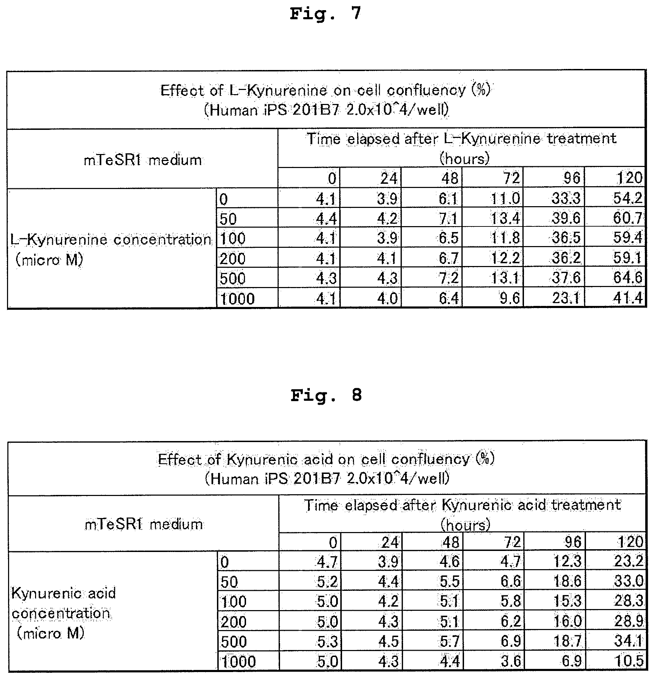

Fig. 7 shows the cell coating rate (%) of human induced pluripotent stem cells 201B7 in mTeSR1 medium supplemented with L-kynurenine to a final concentration of 50 µM, 100 µM, 200 µM, 500 µM or 1000 µM. 201B7 cells were single cell seeded in a 6-well plate at 20,000 cells per well and cultured for 6 days. The cell coating rate was measured at 0, 24, 48, 72, 96 and 120 hr after addition of L-kynurenine (0 hr). The obtained results showed a proliferation promoting effect in L-kynurenine 50-500 µM addition group.
Fig. 8 shows the cell coating rate (%) of human induced pluripotent stem cells 201B7 in mTeSR1 medium supplemented with kynurenic acid to a final concentration of 50 µM, 100 µM, 200 µM, 500 µM or 1000 µM. 201B7 cells were single cell seeded in a 6-well plate at 20,000 cells per well and cultured for 6 days. The cell coating rate was measured at 0, 24, 48, 72, 96 and 120 hr after addition of kynurenic acid (0 hr). The obtained results showed a proliferation promoting effect in kynurenic acid 50-500 µM addition group.

### [Description of Embodiments]

The present invention provides a use of a medium that promotes cell proliferation (hereinafter to be also referred to as the medium of the present invention), a method that promotes cell proliferation (hereinafter to be also referred to as the method of the present invention), and a use of a medium additive for promoting cell proliferation.

### (1) L-tryptophan or L-tryptophan derivative

L-tryptophan (2-amino-3-(indolyl)propionic acid) is one kind of essential amino acid constituting proteins.

In the present specification, L-tryptophan encompasses a salt of L-tryptophan. Examples of the salt of L-tryptophan include, but are not limited to, inorganic acid salts such as hydrochloride, hydrobromide, sulfate, hydroiodide, nitrate salt and phosphate, organic acid salts such as citrate, oxalate, acetate, formate, propionate, benzoate, trifluoroacetate, maleate, tartrate, methanesulfonate, benzenesulfonate and paratoluenesulfonate; inorganic base salts such as sodium salt, potassium salt, calcium salt, magnesium salt and ammonium salt, organic base salts such as triethylammonium salt, triethanol ammonium salt, pyridinium salt and diisopropylammonium salt; amino acid salts such as arginine, aspartic acid and glutamic acid and the like.

As the salt of L-tryptophan, hydrochloride, sodium salt or potassium salt is preferably used.

L-tryptophan can be obtained by a method known per se. Examples of the production method of L-tryptophan include, but are not limited to, the methods described in JP-A-2012-223092, JP-A-2012-100537, JP-A-2011-167071, JP-A-2010-263790, JP-A-2010-110217 and the like.

In addition, commercially available L-tryptophan can also be used. Examples of the commercially available L-tryptophan include, but are not limited to, Wako Pure Chemical Industries, Ltd. 038-23581 (model number), Tokyo Chemical Industry Co., Ltd. T0541 (model number), Nacalai Tesque 13043-92 (model number), MP Biomedicals ICN1031505 (model number), Sigma-Aldrich T8941 (model number) and the like.

The L-tryptophan derivative is not particularly limited as long as it provides L-tryptophan in the medium; for example, a substance that provides L-tryptophan by hydrolysis when added to the medium and the like can be mentioned. Examples of the L-tryptophan derivative include, but are not limited to, dipeptide in which tryptophan and amino acid are peptide-bonded, C1-6 alkylester of tryptophan, N acetyltryptophan and the like.

Examples of the dipeptide in which tryptophan and amino acid are bonded include, but are not limited to, L-alanyl-L-tryptophan, L-arginyl-L-tryptophan, L-asparaginyl-L-tryptophan, L-aspartic acid-L-tryptophan, L-cysteinyl-L-tryptophan, L-glutaminyl-L-tryptophan, L-glutamic acid-L-tryptophan, glycyl-L-tryptophan, L-histidinyl-L-tryptophan, L-isoleucyl-L-tryptophan, L-leucyl-L-tryptophan, L-lysyl-L-tryptophan, L-methionyl-L-tryptophan, L-phenylalanyl-L-tryptophan, L-prolyl-L-tryptophan, L-seryl-L-tryptophan, L-threonyl-L-tryptophan, L-tyrosyl-L-tryptophan, L-valyl-L-tryptophan, L-tryptophanyl-L-tryptophan, L-tryptophanyl-L-alanine, L-tryptophanyl-L-arginine, L-tryptophanyl-L-asparagine, L-tryptophanyl-L-aspartic acid, L-tryptophanyl-L-cysteine, L-tryptophanyl-L-glutamine, L-tryptophanyl-L-glutamic acid, L-tryptophanyl-glycine, L-tryptophanyl-L-histidine, L-tryptophanyl-L-isoleucine, L-tryptophanyl-L-leucine, L-tryptophanyl-L-lysine, L-tryptophanyl-L-methionine, L-tryptophanyl-L-phenylalanine, L-tryptophanyl-L-proline, L-tryptophanyl-L-serine, L-tryptophanyl-L-threonine, L-tryptophanyl-L-tyrosine, L-tryptophanyl-L-valine and the like.

Examples of the C1-6 alkylester of tryptophan include, but are not limited to, L-tryptophanmethylester, L-tryptophanethylester and the like.

In the present specification, the L-tryptophan derivative encompasses salts of L-tryptophan derivative. As the salt of L-tryptophan derivative, those recited above as the salt of L-tryptophan can be mentioned.

When used for culturing pluripotent stem cells, the L-tryptophan derivative is preferably L-alanyl-L-tryptophan or glycyl-L-tryptophan.

In the present specification, the L-tryptophan derivative encompasses a metabolite or a salt of L-tryptophan. When used for culturing pluripotent stem cells, the L-tryptophan metabolite is preferably L-kynurenine or kynurenic acid.

The L-tryptophan derivative can be obtained by a method known per se. For example, the production method of L-tryptophandipeptide includes, but is not limited to, a general solid phase synthetic process and the like.

In addition, commercially available L-tryptophan derivatives can also be used. Examples of the commercially available L-tryptophan derivative include, but are not limited to, Wako Pure Chemical Industries, Ltd. 038-23581 (model number), Tokyo Chemical Industry Co., Ltd. T0541 (model number), Nacalai Tesque 13043-92 (model number), MP Biomedicals ICN1031505 (model number), Sigma-Aldrich T8941 (model number) and the like.

### (2) Pluripotent stem cell

In the present specification, a pluripotent stem cell means an immature cell having self-replication competence and differentiation/proliferative capacity, which is a cell capable of differentiating into any tissue or cell constituting living organisms. Examples of the pluripotent stem cell include embryonic stem cell (ES cell), induced pluripotent stem cell (iPS cell) (Takahashi K et al, Cell. 2007 Nov 30; 131(5): 861-72), spermatogonial stem cell (mGS cell) (Kanatsu-Shinohara M et al., Biol Reprod. 2007 Jan; 76(1): 55-62), embryonic germ cell (Matsui Y et al, Cell. 1992 Sep 4; 70(5): 841-7) and the like.

The pluripotent stem cell can be obtained by a method known per se. For example, non-human embryonic stem cell (ES cell) can be obtained by a method for culturing an inner cell mass in the blastocyst of mammals (e.g., the method described in Manipulating the Mouse Embryo: A Laboratory Manual, Fourth Edition 2014 Cold Spring Harbor Laboratory Press), a method for culturing early embryo produced by somatic cell nuclear transfer (Wilmut et al., Nature. 1997 Feb 27; 385(6619):810-3, Wakayama et al., Nature. 1998 Jul 23; 394(6691):369-74, Wakayama T et al., Science. 2001 Apr 27; 292(5517):740-3) or the like, though the method is not limited to these.

Furthermore, embryonic stem cells can also be obtained from certain institutions. For reference example, KhES-1 cell, KhES-2 cell and KhES-3 cell, which are human ES cells, are available from the Institute of Regenerative Medicine, Kyoto University.

Examples of the method for obtaining induced pluripotent stem cell include, but are not limited to, a method including introduction of a nuclear reprogramming substance (e.g., Oct3/4, Sox2, c-Myc and Klf4 and the like) into somatic cell (Takahashi K et al., Cell. 2006 Aug 25; 126(4):663-76, WO 2007/069666). In addition, induced pluripotent stem cell can be produced according to the methods described in Takahashi K et al., Nat Protoc. 2007; 2(12):3081-9, Aoi et al., Science. 2008 Aug 1; 321(5889):699-702, Takahashi, K et al., Cell. 2007 Nov 30; 131(5):861-72, Yu, J et al., Science. 2007 Dec 21; 318(5858):1917-20, Nakagawa, M et al., Nat Biotechnol. 2008 Jan; 26(1):101-6, and the like. However, the method is not limited to these.

Furthermore, induced pluripotent stem cells can also be obtained from certain institutions. For example, 253G1 cell and 201B7 cell, which are human iPS cells, can be purchased from iPS Academia Japan, Inc..

Embryonic germ cells can be induced by isolating primordial germ cells according to a conventional method and culturing same in the presence of LIF, bFGF and SCF. mGS cells can be produced from testis cells according to the method described in WO 2005/100548.

The pluripotent stem cell used in the present invention is preferably an embryonic stem cell or induced pluripotent stem cell, more preferably an induced pluripotent stem cell.

In the present invention, pluripotent stem cells derived from mammals are generally used. Examples of the mammals include, but are not limited to, rodents such as mouse, rat, hamster, guinea pig and the like, lagomorpha such as rabbit and the like, ungulata such as swine, bovine, goat, horse, sheep and the like, carnivora such as canine, feline and the like, primates such as human, monkey, cynomolgus monkey, marmoset, orangutan, chimpanzee and the like, and the like. In the present invention, pluripotent stem cells derived from rodents such as mouse and the like or primates such as human and the like are preferably used and pluripotent stem cells derived from human are more preferably used.

In the present invention, human induced pluripotent stem cell is most preferably used.

### (3) Medium for culturing pluripotent stem cell

In one embodiment of the present invention, the present invention provides a medium for culturing pluripotent stem cells, containing L-tryptophan or an L-tryptophan derivative at a high concentration (to be also referred to as the medium of the present invention in the present specification). Using the medium of the present invention, pluripotent stem cells can be efficiently proliferated. Particularly, the medium of the present invention is useful for proliferating and maintaining pluripotent stem cells while maintaining an undifferentiated state.

The medium of the present invention contains L-tryptophan or an L-tryptophan derivative at a high concentration of not less than 176 µM. The L-tryptophan concentration of 44 µM corresponds to the concentration of free L-tryptophan in human blood. When pluripotent stem cells are cultured in a general medium containing L-tryptophan corresponding to the concentration of free L-tryptophan in human blood, proliferation of the pluripotent stem cells is limited by early depletion of L-tryptophan in the medium. Since the medium of the present invention contains a high concentration of L-tryptophan or an L-tryptophan derivative, L-tryptophan is hardly depleted during culture of pluripotent stem cells, and a high proliferation rate of pluripotent stem cells and long-term proliferation of pluripotent stem cells can be achieved.

The concentration of L-tryptophan or L-tryptophan not less than 176 µM, preferably not less than 352 µM, further preferably not less than 704 µM. The upper limit of the concentration of L-tryptophan or L-tryptophan derivative in the medium of the present invention is theoretically the saturation concentration of L-tryptophan or L-tryptophan derivative. From the aspects of the solubility of L-tryptophan or L-tryptophan derivative in the medium and the cost, the concentration of L-tryptophan or L-tryptophan derivative in the medium is preferably not more than 1408 µM.

The medium of the present invention has an effect of promoting proliferation of pluripotent stem cells. "Promoting proliferation of pluripotent stem cells" means that the proliferation of pluripotent stem cells is promoted when cultured in the medium of the present invention compared to when cultured in a control medium having the same composition as that of the medium of the present invention except that the concentration of L-tryptophan corresponds to the concentration of free L-tryptophan in human blood (44 µM).

The components other than L-tryptophan or L-tryptophan derivative contained in the medium of the present invention are not particularly limited as long as the effect of promoting proliferation of pluripotent stem cells can be achieved, and the composition used for general maintenance culture of pluripotent stem cells can be employed as appropriate.

The medium of the present invention can be prepared by adding L-tryptophan or an L-tryptophan derivative to the above-mentioned concentration in a medium capable of maintaining pluripotent stem cells. For the production of the medium, one kind of L-tryptophan or L-tryptophan derivative may be used, or plural kinds of L-tryptophan and/or L-tryptophan derivatives may be used in combination.

The medium of the present invention may be prepared using the medium generally used for culturing mammalian cells as a basal medium. The basal medium is not particularly limited as long as the desired effects such as promotion of proliferation of pluripotent stem cells and the like can be achieved. For example, media generally used for culturing animal cells such as BME medium, BGJb medium, CMRL 1066 medium, Glasgow MEM medium, Improved MEM Zinc Option medium, IMDM medium, Medium 199 medium, Eagle MEM medium, αMEM medium, DMEM medium, F-12 medium, DMEM/F12 medium, IMDM/F12 medium, ham medium, RPMI 1640 medium, Fischer's medium, or mixed medium of these and the like can be mentioned. The medium of the present invention may be prepared using the medium generally used for culturing pluripotent stem cells as a basal medium. As a commercially available basal medium for culturing stem cells, StemFit (registered trade mark) AK medium (Ajinomoto Co., Inc.), Essential 8 medium (Life Technologies Inc.), mTeSR1 medium (STEMCELL Technologies Inc.), TeSR2 medium (STEMCELL Technologies Inc.), RHB medium (StemCells, Inc.), TeSRTM-E6 (STEMCELL Technologies Inc.), hESF-GRO medium (NIPRO CORPORATION), HESF-DIF medium (NIPRO CORPORATION), CSTI-7 (Cell Science & Technology Institute, Inc.), Essential 6 medium (Life Technologies Inc.) and the like can be mentioned

To avoid contamination with chemically-undefined components, the medium of the present invention is preferably a medium containing chemically-defined components (Chemically defined medium; CDM). To avoid contamination with chemically-undefined components, the medium of the present invention is preferably a serum-free medium. The "serum-free medium" in the present invention means a medium free of unconditioned or unpurified serum. In the present invention, media containing purified components derived from blood or animal tissues (e.g., growth factors such as bFGF and the like) are also included in the serum-free medium as long as unconditioned or unpurified serum is not contained.

The serum-free medium may contain a serum replacement. Examples of the serum replacement include those appropriately containing serum albumin, transferrin, fatty acid, collagen precursor, trace element, 2-mercaptoethanol, 3'thiolglycerol, equivalents of these and the like. Such serum replacement can be prepared by the method described in, for example, WO 98/30679. As the serum replacement, a commercially available product may also be utilized. Examples of such commercially available serum replacement include, but are not limited to, Knockout^{™} Serum Replacement (Life Technologies Inc.: hereinafter sometimes to be also indicated as KSR), Chemically-defined Lipid concentrate (Life Technologies Inc.), Glutamax^{™} (Life Technologies Inc.), B27 (Life Technologies Inc.), and N2 (Life Technologies Inc.).

Generally, the medium of the present invention contains, in addition to L-tryptophan, all essential amino acids (L-leucine, L-lysine, L-phenylalanine, L-isoleucine, L-threonine, L-histidine, L-methionine and L-valine) other than L-tryptophan.

The medium of the present invention preferably contains all non-essential amino acids (L-alanine, L-arginine, L-asparagine, L-aspartic acid, glycine, L-glutamine, L-glutamic acid, L-cysteine, L-serine, L-tyrosine, L-proline). L-alanyl-L-glutamine may also be used instead of L-glutamine.

The medium of the present invention may contain natural amino acids such as L-cystine and the like in addition to the aforementioned 20 kinds of amino acids.

The medium of the present invention may further contain a medium additive. Examples of the medium additive include, but are not limited to, ROCK (Rho-associated coiled-coil forming kinase/Rho binding kinase) inhibitor such as Y-27632 and the like, antibiotics such as penicillin-streptomycin and the like, vitamins, L-ascorbic acid, magnesium L-ascorbyl phosphate, sodium pyruvate, 2-aminoethanol, glucose, sodium hydrogen carbonate, HEPES, insulin, progesterone, sodium selenate, putrescine and the like. Additives are preferably contained in a known concentration range.

The medium of the present invention may contain a fatty acid. Examples of the fatty acid to be contained in the medium of the present invention include, but are not limited to, oleic acid, linoleic acid, α-linolenic acid, γ-linolenic acid, palmitic acid, stearic acid, arachidonic acid, icosapentaenoic acid, docosahexaenoic acid, butyric acid, acetic acid, pulmitoleic acid, valeric acid (valerianic acid), caproic acid, enanthic acid (hepthylic acid), caprylic acid, pelargric acid, capric acid, lauric acid, myristic acid, pentadecylic acid, margaric acid, Khusenic acid, eleostearic acid, arachidic acid, 8,11- eicosadienoic acid, 5,8,11-eicosatrienoic, behenic acid, lignoceric acid, nervonic acid, cerotic acid, montanic acid, melissic acid and the like. The fatty acid to be contained in the medium of the present invention may be saturated fatty acid or unsaturated fatty acid.

A composition used for known cell culture may be appropriately employed for the medium of the present invention according to the purpose of use. For example, when proliferation while maintaining an undifferentiated state of pluripotent stem cells is intended, the medium of the present invention preferably does not contain a substance having an effect of promoting differentiation of pluripotent stem cells but preferably contains a substance having an effect of suppressing differentiation of pluripotent stem cells. Examples of the substance having an effect of suppressing differentiation of pluripotent stem cells include FGF2 and the like for human pluripotent stem cells, and leukemia inhibitory factor (LIF) and the like for mouse pluripotent stem cells.

More specifically, examples of the medium for promoting proliferation while maintaining an undifferentiated state of pluripotent stem cells include a medium obtained by adding L-ascorbic acid, selenium, transferrin, NaHCO₃, insulin, FGF2 and TGFβ1 to DMEM/F-12 medium (Chen G et al., Nat Methods. 2011 May; 8(5):424-429), a medium obtained by adding non-essential amino acid, L-glutamine, β-mercaptoethanol, insulin, transferrin, cholesterol, serum albumin, pipecolic acid, lithium chloride, FGF2 and TGFβ1 to DMEM/F-12 medium (Ludwig TE et al., Nat Methods. 2006 Aug; 3(8):637-46), serum-free medium for maintaining mouse embryonic stem cells and added with leukemia inhibitory factor, poly(vinyl alcohol), L-glutamine, insulin, transferrin, selenium, 2-mercaptoethanol and antibiotic (JP-A-2007-228815), serum-free medium obtained by mixing pannexin, bFGF, PDGF, EGF and vitamin C (JP-A-2008-148643), a medium for maintaining multipotency of mesenchymal stem cells and characteristically containing TGF-β (JP-A-2010-094062) and the like. The medium of the present invention can be prepared by reference to these compositions.

For example, the medium of the present invention is prepared by adding L-tryptophan or an L-tryptophan derivative to a final concentration of not less than 176 µM in a basal medium containing L-ascorbic acid, selenium, transferrin, insulin, FGF2 and TGFβ1. However, the medium is not limited to this.

The pH of the medium of the present invention is preferably about 6.0 - about 8.5, more preferably adjusted to about 7.0 - about 7.5. The medium is preferably subjected to a sterilization treatment such as filtration sterilization using a membrane filter or the like.

The medium of the present invention can be used for any culture method such as adhesion culture, suspension culture, embedded culture, tissue culture and the like.

The form of the medium of the present invention is not particularly limited as long as the desired effect of the present invention is obtained. For example, it can be prepared in the form of a liquid medium, a semi-fluid medium or a solid medium. The medium of the present invention may be prepared in a powder form. By preparing in a powder form, transportation and storage may be extremely facilitated. In addition, a liquid, semi-liquid or solid medium can be easily prepared by adding sterilized water and/or agar and the like when in use.

### (4) Culture method 1 of pluripotent stem cell

The present invention provides a method for culturing a pluripotent stem cell including culturing the pluripotency stem cell in the above-mentioned medium of the present invention (to be also referred to as the method 1 of the present invention in the present specification).

Using the medium of the present invention, pluripotent stem cells can be efficiently proliferated. Particularly, the medium of the present invention is useful for proliferating and maintaining pluripotent stem cells while maintaining an undifferentiated state. Therefore, the method 1 of the present invention is preferably a method for proliferating pluripotent stem cells, more preferably a method for proliferating or maintaining pluripotent stem cells while maintaining an undifferentiated state.

In the method 1 of the present invention, the concentration of the pluripotent stem cells in the medium is not particularly limited as long as the desired effect is obtained. It is generally 10⁰ - 10⁷ cells/cm³, preferably 10¹ - 10⁶ cells/cm³, more preferably 10² - 10⁵ cells/cm³.

The pluripotent stem cells may be cultured by seeding pluripotent stem cells in the above-mentioned medium of the present invention in which the concentration of L-tryptophan or L-tryptophan derivative is adjusted in advance to a desired concentration. Alternatively, the pluripotent stem cells may be cultured by adding L-tryptophan or L-tryptophan derivative to a medium after start of cell culture, adjusting the concentration of the L-tryptophan or L-tryptophan derivative to a concentration required by the medium of the present invention, and further continuing the culture.

When L-tryptophan or L-tryptophan derivative is added after start of the cell culture, the medium additive of the present invention described in detail below may also be used.

The culture conditions in the method 1 of the present invention are not particularly limited as long as the desired effects such as promotion of proliferation of pluripotent stem cells and the like can be achieved, except that the medium of the present invention is used, and culture conditions generally used for culturing pluripotent stem cells can be appropriately employed according to the object of culture.

For example, as a method for culturing pluripotent stem cells while maintaining an undifferentiated state, the methods described in Experimental Medicine separate volume, Cell Culture Protocol to be Selected by Objective (YODOSHA CO., LTD.) and the like can be mentioned. Pluripotent stem cells can be cultured using feeder cells such as mouse embryo fibroblast (MEF), mouse fibroblast cell line (STO) and the like, or under a feeder-free environment.

In the method 1 of the present invention, an incubator to be used for culturing cells is not particularly limited as long as cells can be cultured. Examples thereof include flask, tissue culture flask, dish, petri dish, tissue culture dish, multidish, microplate, microwell plate, multiplate, multiwell plate, microslide, chamber slide, schale, tube, tray, culture bag, and roller bottle.

An incubator used for culturing cells may be cell adhesive or cell non-adhesive, and is appropriately selected according to the object.

A cell adhesive incubator may be coated with any cell supporting substrate such as extracellular matrix (ECM) and the like or an artificial material mimicking the function thereof, for the purpose of improving the adhesiveness of the cells to the surface of the incubator. The cell supporting substrate may be any substance aiming at adhesion of stem cells or feeder cells (when used).

Other culture conditions can be appropriately determined. For example, the culture temperature is not particularly limited as long as the desired effects such as promotion of proliferation of cells and the like can be achieved. It is about 30 - 40°C, preferably about 37°C. The CO₂ concentration is about 1 - 10%, preferably about 2 - 5%. The oxygen concentration is generally 1 - 40%, and is appropriately selected according to culture conditions and the like.

In the method 1 of the present invention, pluripotent stem cells can be cultured by a method known per se such as adhesion culture, suspension culture, tissue culture and the like.

While the period for culturing pluripotent stem cells in the method 1 of the present invention is not particularly limited as long as the desired effects such as promotion of cell proliferation and the like can be achieved, it is generally not less than 2 days, preferably not less than 4 days, more preferably not less than 7 days. Theoretically, culture can be continued infinitely. The pluripotent stem cells cultured in the medium of the present invention are collected, a part or all of them are passaged in the fresh medium of the present invention, and the culture is continued, whereby the pluripotent stem cells can be proliferated or maintained while maintaining an undifferentiated state over a long period of time.

### (5) Pluripotent stem cell culture preparation

The present invention provides a pluripotent stem cell culture preparation containing the above-mentioned medium of the present invention and pluripotent stem cells (the culture preparation of the present invention).

The pluripotent stem cell in the culture preparation of the present invention is a living and proliferating cell.

It is preferable that the pluripotent stem cell in the culture preparation of the present invention be isolated. Being "isolated" means that an operation to remove factors other than the object components and cells has been performed and thus they are no longer in a naturally occurring state. The purity of the "isolated pluripotent stem cells" (percentage of the number of pluripotent stem cells in the total number of cells) is generally not less than 70%, preferably not less than 80%, more preferably not less than 90%, further preferably not less than 99%, most preferably 100%.

In the culture preparation of the present invention, the pluripotent stem cell is present in, for example, a liquid or semi-fluid medium of the present invention. In one embodiment, the culture preparation of the present invention is a suspension of pluripotent stem cells in the medium of the present invention. The culture preparation of the present invention may be sealed in an appropriate container.

The culture preparation of the present invention is useful in practicing the above-mentioned method 1 of the present invention.

### (6) Culture method 2 of pluripotent stem cell

A method for culturing a pluripotent stem cell including the following steps (the culture method 2 of the present invention) :
(1) culturing a pluripotent stem cell in a medium comprising L-tryptophan or an L-tryptophan derivative;
(2) adding L-tryptophan or an L-tryptophan derivative to the obtained pluripotent stem cell culture to supplement a part or all of the L-tryptophan or L-tryptophan derivative in the medium and consumed in (1); and
(3) successively culturing the pluripotent stem cell culture added with the L-tryptophan or L-tryptophan derivative.

The medium of the present invention may be used in step (1). However, the concentration of L-tryptophan or L-tryptophan derivative in the medium used in step (1) only needs to be a concentration at which pluripotent stem cells can be proliferated (preferably a concentration at which pluripotent stem cells can be proliferated and maintained while maintaining an undifferentiated state) and does not need to be a "high concentration" as in the medium of the present invention. The concentration of L-tryptophan or L-tryptophan derivative in the medium used in step (1) is, for example, not less than 10 µM, preferably not less than 15 µM, more preferably not less than 44 µM, at the time point when culture is started.

The composition of the medium used in step (1) is the same as that of the medium of the present invention except that the concentration of L-tryptophan or L-tryptophan derivative does not need to be a "high concentration".

The culture conditions in step (1) are the same as those of the above-mentioned method 1 of the present invention except that the concentration of L-tryptophan or L-tryptophan derivative does not need to be a "high concentration".

As a result of the culture in step (1), pluripotent stem cells proliferate (preferably proliferate while maintaining an undifferentiated state) along with which L-tryptophan or L-tryptophan derivative in the medium is consumed and the concentration thereof in the medium decreases.

In step (2), the timing of adding L-tryptophan or an L-tryptophan derivative to the pluripotent stem cell culture obtained in step (1) is not particularly limited as long as the desired effect such as promotion of proliferation of pluripotent stem cells and the like can be achieved, and it can be added at any timing. For example, in step (1), L-tryptophan or L-tryptophan derivative is added when the concentration of L-tryptophan or L-tryptophan derivative in the medium decreases to less than 10 µM, preferably less than 15 µM, more preferably less than 44 µM. Alternatively, in the step (1), L-tryptophan or L-tryptophan derivative is added when the concentration of L-tryptophan or L-tryptophan derivative in the medium decreases to not more than 50%, preferably not more than 25%, from 100% at the start of culture. For example, L-tryptophan or L-tryptophan derivative can be added after 2 to 5 days, preferably 3 to 5 days, more preferably 4 to 5 days, after the start of culture in step (1).

As L-tryptophan and/or L-tryptophan derivative to be added to the medium, one kind of L-tryptophan or L-tryptophan derivative may be used or plural kinds of L-tryptophan and/or L-tryptophan derivative may be used in combination.

The amount of L-tryptophan or L-tryptophan derivative to be added to the medium is such that the concentration of L-tryptophan or L-tryptophan derivative in the medium is not less than 176 µM, preferably not less than 352 µM. L-tryptophan or L-tryptophan derivative is added such that the concentration thereof in the medium is not less than 176 µM, preferably not less than 352 µM, further preferably not less than 704 µM. The upper limit of the concentration of L-tryptophan or L-tryptophan derivative in the medium after the addition is theoretically the saturation concentration of L-tryptophan or L-tryptophan derivative. From the aspects of the solubility of L-tryptophan or L-tryptophan derivative in the medium and the cost, the concentration of L-tryptophan or L-tryptophan derivative in the medium is preferably not more than 1408 µM.

To "supplement a part or all of the L-tryptophan or L-tryptophan derivative in the medium and consumed in (1)" in the present specification also includes addition of L-tryptophan or L-tryptophan derivative in an amount exceeding the amount added to the initial culture in step (1), in addition to supplementation of a part or all of the amount of L-tryptophan or L-tryptophan derivative added to the initial culture.

Here, the method 2 of the present invention is characterized by, in the culture of pluripotent stem cells, supplementation of a part or all of L-tryptophan consumed and depleted earliest among the amino acids contained in the medium by the addition of L-tryptophan or L-tryptophan derivative from the outside. The amino acids other than L-tryptophan may or may not be added together with L-tryptophan or L-tryptophan derivative.

In one embodiment, L-tryptophan or L-tryptophan derivative alone is added as amino acid in step (2) and other amino acids are not added.

In another embodiment, in step (2), amino acids other than L-tryptophan (L-leucine, L-lysine, L-phenylalanine, L-isoleucine, L-threonine, L-histidine, L-methionine, L-valine, L-alanine, L-arginine, L-asparagine, L-aspartic acid, glycine, L-glutamine, L-glutamic acid, L-cysteine, L-serine, L-tyrosine, L-proline) or a derivative thereof may or may not be added together with L-tryptophan or L-tryptophan derivative. One kind or plural kinds of amino acids may be added.

In step (3), the pluripotent stem cell culture added with L-tryptophan or L-tryptophan derivative is successively cultured. The culture conditions in step (3) may be the same as those in step (1) or may be changed as long as the desired effects of the present invention are achieved. In one preferable embodiment of the present invention, the culture conditions in step (3) are the same as those in step (1). The addition of L-tryptophan or L-tryptophan derivative avoids depletion of L-tryptophan. Therefore, pluripotent stem cells can continue to proliferate (preferably, while maintaining an undifferentiated state).

In the method 2 of the present invention, replacement of the whole medium is not required, and addition of, as amino acid, L-tryptophan or L-tryptophan derivative alone or only a part of amino acid including L-tryptophan or L-tryptophan derivative can maintain proliferation of pluripotent stem cells. Thus, pluripotent stem cells can be proliferated at a low cost and efficiently.

### (7) Medium additive

The present invention provides medium additives containing L-tryptophan or L-tryptophan derivative (to be also referred to as the medium additive of the present invention in the present specification). The medium additive of the present invention can be used when L-tryptophan or L-tryptophan derivative is added in the above-mentioned method 1 or 2 of the present invention.

As L-tryptophan and/or L-tryptophan derivative to be contained in the medium additive of the present invention, one kind of L-tryptophan or L-tryptophan derivative may be used or plural kinds of L-tryptophan and/or L-tryptophan derivative may be used in combination.

Addition of the medium additive of the present invention to the culture medium of pluripotent stem cells provides the effect of promoting proliferation of pluripotent stem cells. The medium additive of the present invention is preferably for promoting proliferation of pluripotent stem cells.

The medium additive of the present invention can further contain, in addition to L-tryptophan and/or L-tryptophan derivative, serum replacement, medium additive and fatty acid according to the object of use as long as the desired effect is not impaired. These serum replacement, medium additive and fatty acid are as described above and each is preferably contained within a concentration range known per se. The medium additive of the present invention can further contain, in addition to L-tryptophan and/or L-tryptophan derivative, additives conventionally used for culturing cells and the like as appropriate as long as the desired effect is not impaired.

In one embodiment, the medium additive of the present invention contains L-tryptophan or L-tryptophan derivative alone as amino acid, and does not contain other amino acids.

In one embodiment, the medium additive of the present invention contains, in addition to L-tryptophan or L-tryptophan derivative, 1, 2, 3, 4, 5 or 6 kinds of amino acids selected from the group consisting of L-glutamine, L-arginine, L-cysteine, L-aspartic acid, L-serine and L-methionine. In this case, amino acids other than the aforementioned amino acids may or may not be contained in the medium additive of the present invention as the amino acid contained in the medium additive of the present invention.

The medium additive of the present invention may further contain, in addition to L-tryptophan or L-tryptophan derivative, an adequate amount of any additive, for example, stabilizer, isotonicity agent, pH adjuster and the like as long as the desired effect is not impaired.

The medium additive of the present invention may take any dosage form as long as the desired effect is obtained and, for example, solution, solid, powder and the like can be mentioned. When it is a solid or powder, it is dissolved in an appropriate buffer and the like to a desired concentration, and can be used.

When the medium additive is a solution, the pH of the solution is preferably about 5.0 - about 8.5, and more preferably adjusted to about 6.0 - about 8.0. When the medium additive is a solution, the solution is preferably subjected to a sterilization treatment such as sterilization by filtration using a membrane filter and the like, and the like.

The present invention is explained in more detail in the following by referring to Reference Example and Examples, which do not limit the scope of the present invention.

### (Reference Example)

Using a medium for which the amount of each amino acid therein was quantified in advance, pluripotent stem cell 201B7 strain (iPS Academia Japan, Inc.) was cultured for 5 days and the amount of each amino acid in the medium was measured. The pluripotent stem cells were cultured by seeding 1,000,000 cells in a 100 mm tissue culture dish (353003, Japan Becton, Dickinson and Company) coated with Matrigel (354277, Corning Incorporated) and culturing at 5% CO₂/37°C. In addition, the amount of the amino acid remaining in the medium was measured by the following method. The quantitative analysis of the amino acid was performed by the LC-MS/MS system reported in Shimbo et al. (Anal Chem. 2009 Jul 1; 81(13):5172-9. Multifunctional and highly sensitive precolumn reagents for amino acids in liquid chromatography/tandem mass spectrometry. Shimbo K, Yahashi A, Hirayama K, Nakazawa M, Miyano H.). After cell culture, the supernatant was taken in a 1.5 mL tube and preserved at -80°C until measurement. The sample was subjected to a protein removal treatment, derivatized with an APDS reagent and subjected to an analysis apparatus. The concentration of the amino acid in each sample was calculated using an analytical curve. As a result, L-tryptophan contained at a concentration of 44 µM at the start of culture was depleted on day 4 of the culture. On the other hand, other amino acids remained in the medium even after 5 days of culture, and at least about 20% of the remaining amino acids were confirmed. Therefore, it was found that L-tryptophan is depleted earliest in all amino acids in culturing pluripotent stem cells.

### [Examples]

### Example 1: Proliferation promoting effect of L-tryptophan in commercially available 3 kinds of media

First, the proliferation promoting effect of L-tryptophan on induced pluripotent stem cells (iPS cells) was evaluated using 3 kinds of commercially available media. As the human iPS cell, 201B7 strain purchased from iPS Academia Japan, Inc. was used and cultured under the conditions of 5% CO₂/37°C.

L-tryptophan (Sigma-Aldrich Co. LLC: T8941) was added to the media of Essential-8 (Life Technologies Inc.: A1517001), mTeSR1 (STEM CELL Technologies Inc.: 05850), TeSR2 (STEM CELL Technologies Inc.: 05860) at a given concentration, used for culture and the effect thereof was examined.

L-tryptophan (Sigma-Aldrich Co. LLC: T8941) was added at a final concentration of 44, 176, 352, 704, 1408 µM to Essential-8, mTeSR1, TeSR2 media and the proliferation promoting effect of L-tryptophan was examined using the prepared media. A 6 well plate coated with Matrigel (Japan Becton, Dickinson and Company) as a basement membrane matrix was prepared and 13,000 cells were single cell seeded per well. The next day of cell seeding, evaluation was performed using the medium prepared as mentioned above. The culture period was set to 6 days, the time of L-tryptophan addition was set to 0 and the cell coating rate was measured using IncuCyte at 24, 48, 72, 96 and 120 hr later. As the medium to be used when seeding, a medium without addition of Y-27632 at a final concentration of 10 µM was used and culturing was performed.

The results of a series of experiments performed for each medium are shown in Figs. 1, 2, 3. The obtained results showed a proliferation promoting effect in an L-tryptophan concentration dependent manner. Expression of undifferentiated marker (OCT, Nanog, alkaline phosphatase) was not influenced by the addition of a high concentration of L-tryptophan (data not shown).

### Example 2: Proliferation promoting effect of L-tryptophan - culture results using other human induced pluripotent stem cell line

Then, the proliferation promoting effect of L-tryptophan was evaluated using other strain 253G4 of human induced pluripotent stem cell (iPS cell) and human embryonic stem cell H9 strain. As the medium, mTeSR1 (STEM CELL Technologies Inc.: 05850) was used and culturing was performed under 5% CO₂/37°C conditions.

L-tryptophan (Sigma-Aldrich Co. LLC: T8941) was added to mTeSR1 medium (STEM CELL Technologies Inc.: 05850) at a given concentration, used for culture and the effect thereof was examined.

L-tryptophan (Sigma-Aldrich Co. LLC: T8941) was added at a final concentration of 44, 176, 352, 704, 1408 µM to mTeSR1 medium and the proliferation promoting effect of L-tryptophan was examined using the prepared medium. A 6 well plate coated with Matrigel (Japan Becton, Dickinson and Company) as a basement membrane matrix was prepared and 40,000 253G4 cells and 10,000 H9 cells were single cell seeded per well. The next day of cell seeding, evaluation was performed by replacing the medium with the medium prepared as mentioned above. The culture period was set to 6 days, the time of L-tryptophan addition was set to 0 and the cell coating rate was measured using IncuCyte at 24, 48, 72, 96 and 120 hr later. As the medium to be used when seeding, a medium without addition of Y-27632 at a final concentration of 10 µM was used and culturing was performed.

The results of 5 series of experiments performed for each medium are shown in Figs. 4, 5. The concentration-dependent proliferation promoting effect of L-tryptophan was also confirmed in other human pluripotent stem cells.

### Example 3: Proliferation promoting effect of L-tryptophan - culture results using other human embryonic kidney cell line HEK293T

Next, the proliferation promoting effect of L-tryptophan was evaluated using human embryonic kidney cell line HEK293T cells. As the medium, DMEM medium (Thermo Fisher Scientific K.K.: 11965) added with 10% fetal bovine serum was used and culturing was performed under 5% CO₂/37°C conditions.

L-tryptophan (Sigma-Aldrich Co. LLC: T8941) was added at a given concentration to DMEM medium (Thermo Fisher Scientific K.K.: 11965) added with 10% fetal bovine serum, used for culture and the effect thereof was examined.

L-tryptophan (Sigma-Aldrich Co. LLC: T8941) was added at a final concentration of 44, 176, 352, 704, 1408 µM to DMEM medium (Thermo Fisher Scientific K.K.: 11965) added with 10% fetal bovine serum and the proliferation promoting effect of L-tryptophan was examined using the prepared medium. A 6 well plate was prepared and 10,000 cells were single cell seeded per well. The next day of cell seeding, evaluation was performed by replacing the medium with the medium prepared as mentioned above. The culture period was set to 6 days, the time of L-tryptophan addition was set to 0 and the cell coating rate was measured using IncuCyte at 24, 48, 72, 96 and 120 hr later.

The results of 5 series of experiments are shown in Fig. 6. The concentration-dependent proliferation promoting effect of L-tryptophan was not confirmed in human embryonic kidney cell line HEK293 cells.

### Example 4: Proliferation promoting effect of L-kynurenine in commercially available medium

L-kynurenine (Sigma-Aldrich Co. LLC: K8625) was added at a final concentration of 50, 100, 200, 500, 1000 µM to mTeSR1 medium and the proliferation promoting effect was examined using the prepared medium. Y-27632 was added at a final concentration of 10 µM for 2 days after seeding. As a basement membrane matrix, Matrigel (Japan Becton, Dickinson and Company) was applied on a 6 well plate and 20,000 cells were single cell seeded per well. Two days after the cell seeding, evaluation was performed using the medium free of Y-27632 and prepared as mentioned above. The time of L-kynurenine addition was taken as 0 and the cell coating rate was measured using IncuCyte at 0, 24, 48, 72, 96 and 120 hr later. The results are shown in Fig. 7. As shown in Fig. 7, the obtained results showed a cell proliferation promoting effect in the L-kynurenine 50-500 µM addition group.

### Example 5: Proliferation promoting effect of kynurenic acid in commercially available medium

Kynurenic acid (Sigma-Aldrich Co. LLC: K3375) was added at a final concentration of 50, 100, 200, 500, 1000 µM to mTeSR1 medium and the proliferation promoting effect was examined using the prepared medium. Y-27632 was added at a final concentration of 10 µM for 2 days after seeding. As a basement membrane matrix, Matrigel (Japan Becton, Dickinson and Company) was applied on a 6 well plate and 20,000 cells were single cell seeded per well. Two days after the cell seeding, evaluation was performed using the medium free of Y-27632 and prepared as mentioned above. The time of kynurenic acid addition was taken as 0 and the cell coating rate was measured using IncuCyte at 0, 24, 48, 72, 96 and 120 hr later. The results are shown in Fig. 8. As shown in Fig. 8, the obtained results showed a cell proliferation promoting effect in the kynurenic acid 50-500 µM addition group.

### [Industrial Applicability]

According to the present invention, proliferation of pluripotent stem cells can be promoted and human costs and monetary costs for culturing pluripotent stem cells can be reduced.

This application is based on a patent application No. 2017-063842 filed in Japan (filing date: March 28, 2017)

## Claims

1. Use of a serum-free medium for culturing a pluripotent stem cell, wherein the medium comprises L-tryptophan or an L-tryptophan derivative at a concentration of not less than 176 µM.

2. The use of a medium according to claim 1, wherein the concentration of the L-tryptophan or L-tryptophan derivative in the medium is 176 µM - 1408 µM.

3. The use of the medium according to claim 1 or 2, wherein the pluripotent stem cell is an induced pluripotent stem cell.

4. The use of the medium according to any one of claims 1 to 3, wherein the tryptophan derivative is a dipeptide in which tryptophan and amino acid are peptide-bonded.

5. A method for culturing a pluripotent stem cell comprising culturing the pluripotent stem cell in the medium according to any one of claims 1 to 4.

6. The method according to claim 5 wherein the method is for proliferating a pluripotent stem cell.

7. A pluripotent stem cell culture preparation comprising the medium according to any one of claims 1 to 4 and a pluripotent stem cell.

8. A method for culturing a pluripotent stem cell comprising the following steps:
(1) culturing a pluripotent stem cell in a serum-free medium comprising L-tryptophan or an L-tryptophan derivative;
(2) adding L-tryptophan or an L-tryptophan derivative to the obtained pluripotent stem cell culture such that the concentration of the L-tryptophan or an L-tryptophan derivative in the medium is not less than 176 µM to supplement a part or all of the L-tryptophan or L-tryptophan derivative in the medium and consumed in (1); and
(3) successively culturing the pluripotent stem cell culture added with the L-tryptophan or L-tryptophan derivative.

9. The method according to claim 8 wherein the tryptophan derivative is a dipeptide in which tryptophan and amino acid are peptide-bonded.

10. Use of a serum-free-medium additive for promoting proliferation of a pluripotent stem cell comprising L-tryptophan or an L-tryptophan derivative, wherein the concentration of the L-tryptophan or an L-tryptophan derivative in the serum-free medium is not less than 176 µM.

11. The use of the medium additive according to claim 10, wherein the tryptophan derivative is a dipeptide in which tryptophan and amino acid are peptide-bonded.

## Patentansprüche

1. Verwendung eines serumfreien Mediums zum Kultivieren einer pluripotenten Stammzelle, wobei das Medium L-Tryptophan oder ein L-Tryptophanderivat bei einer Konzentration von nicht weniger als 176 µM umfasst.

2. Verwendung eines Mediums gemäß Anspruch 1, wobei die Konzentration des L-Tryptophans oder des L-Tryptophanderivats in den Medium 176 µM - 1408 µM beträgt.

3. Verwendung des Mediums gemäß Anspruch 1 oder 2, wobei die pluripotente Stammzelle eine induzierte pluripotente Stammzelle ist.

4. Verwendung des Mediums gemäß irgendeinem der Ansprüche 1 bis 3, wobei das Tryptophanderivat ein Dipeptid ist, worin Tryptophan und Aminosäure Peptid-gebunden sind.

5. Verfahren zum Kultivieren einer pluripotenten Stammzelle, umfassend das Kultivieren der pluripotenten Stammzelle in dem Medium gemäß irgendeinem der Ansprüche 1 bis 4.

6. Verfahren gemäß Anspruch 5, wobei das Verfahren zum Proliferieren einer pluripotenten Stammzelle ist.

7. Zubereitung einer Kultur pluripotenter Stammzellen, umfassend das Medium gemäß irgendeinem der Ansprüche 1 bis 4 und eine pluripotente Stammzelle.

8. Verfahren zum Kultivieren einer pluripotenten Stammzelle, umfassend die folgenden Schritte:
(1) Kultivieren einer pluripotenten Stammzelle in einem serumfreien Medium, welches L-Tryptophan oder ein L-Tryptophanderivat umfasst;
(2) Zugeben von L-Tryptophan oder eines L-Tryptophanderivats zu der erhaltenen Kultur pluripotenter Stammzellen, so dass die Konzentration des L-Tryptophans oder eines L-Tryptophanderivats in dem Medium nicht weniger als 176 µM beträgt, um einen Teil des oder das gesamte L-Tryptophan(s) oder L-Tryptophanderivat(s) in dem Medium und verbraucht in (1) zu supplementieren; und
(3) sukzessives Kultivieren der mit dem L-Tryptophan oder dem L-Tryptophanderivat versetzten Kultur pluripotenter Stammzellen.

9. Verfahren gemäß Anspruch 8, wobei das Tryptophanderivat ein Dipeptid ist, worin Tryptophan und Aminosäure Peptid-gebunden sind.

10. Verwendung eines serumfreies-Medium-Additivs zur Förderung der Proliferation einer pluripotenten Stammzelle, umfassend L-Tryptophan oder ein L-Tryptophanderivat, wobei die Konzentration des L-Tryptophans oder eines L-Tryptophanderivats in dem serumfreien Medium nicht weniger als 176 µM beträgt.

11. Verwendung des Medium-Additivs gemäß Anspruch 10, wobei das Tryptophanderivat ein Dipeptid ist, worin Tryptophan und Aminosäure Peptid-gebunden sind.

## Revendications

1. Utilisation d'un milieu sans sérum pour cultiver une cellule souche pluripotente, dans laquelle le milieu comprend du L-tryptophane ou un dérivé de L-tryptophane à une concentration non inférieure à 176 µM.

2. Utilisation d'un milieu selon la revendication 1, dans laquelle la concentration du L-tryptophane ou du dérivé de L-tryptophane dans le milieu est de 176 µM à 1 408 µM.

3. Utilisation d'un milieu selon la revendication 1 ou 2, dans laquelle la cellule souche pluripotente est une cellule souche pluripotente induite.

4. Utilisation d'un milieu selon l'une quelconque des revendications 1 à 3, dans laquelle le dérivé de tryptophane est un dipeptide dans lequel du tryptophane et un acide aminé sont liés par liaison peptidique.

5. Procédé pour cultiver une cellule souche pluripotente, comprenant la culture de la cellule souche pluripotente dans le milieu de l'une quelconque des revendications 1 à 4.

6. Procédé selon la revendication 5, lequel procédé est pour la prolifération d'une cellule souche pluripotente.

7. Préparation de culture de cellule souche pluripotente comprenant le milieu de l'une quelconque des revendications 1 à 4 et une cellule souche pluripotente.

8. Procédé pour cultiver une cellule souche pluripotente, comprenant les étapes suivantes :
(1) culture d'une cellule souche pluripotente dans un milieu sans sérum comprenant du L-tryptophane ou un dérivé de L-tryptophane ;
(2) addition de L-tryptophane ou d'un dérivé de L-tryptophane à la culture de cellule souche pluripotente obtenue de façon que la concentration du L-tryptophane ou du dérivé de L-tryptophane dans le milieu ne soit pas inférieure à 176 µM pour supplémenter tout ou partie du L-tryptophane ou du dérivé de L-tryptophane dans le milieu et consommé dans l'étape (1) ; et
(3) ensuite culture de la culture de cellule souche pluripotente additionnée du L-tryptophane ou du dérivé de L-tryptophane.

9. Procédé selon la revendication 8, dans lequel le dérivé de tryptophane est un dipeptide dans lequel du tryptophane et un acide aminé sont liés par liaison peptidique.

10. Utilisation d'un additif de milieu sans sérum pour promouvoir la prolifération d'une cellule souche pluripotente comprenant du L-tryptophane ou un dérivé de L-tryptophane, dans laquelle la concentration du L-tryptophane ou du dérivé de L-tryptophane dans le milieu sans sérum n'est pas inférieure à 176 µM.

11. Utilisation d'un additif de milieu selon la revendication 10, dans laquelle le dérivé de tryptophane est un dipeptide dans lequel du tryptophane et un acide aminé sont liés par liaison peptidique.
